# EUROPEAN PATENT APPLICATION

(11) **EP 2 151 501 A1**
(43) Date of publication of application: **10.02.2010**
(21) Application number: 08104902.5
(22) Date of filing: 28.07.2008
(51) Int. Cl.: C12Q 1/24, G01N 33/569, C12N 1/02, C12M 1/26

(54) **Method and device for the enrichment of biological material**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The method according to the present invention is suitable for enriching biological material, like e.g. bacteria or DNA. The corresponding microfluidic device used in the inventive method has at least one conducting polymer electrode and an electrolyte. The enrichment is achieved by applying an electrical voltage to the at least one electrode.

## Description

### FIELD OF THE INVENTION

The present invention relates to a method and a device for the enrichment of biological material using a polymer electrode.

### BACKGROUND OF THE INVENTION

Patients with life threatening infectious diseases are an ever-increasing problem in hospital environments. This is most problematic for patients in e.g. intensive care units. With the emergence of antibiotic-resistant bacterial strains such as methillicin-resistant *Staphylococcus aureus* or ciprofloxacin-resistant *Escherichia coli* (*E coli*) also other patients who are not even in the Intensive Care Unit are at risk. In all cases, life-threatening infections require fast and accurate diagnosis. For infectious diseases the question whether the corresponding bacteria can be treated with a specific antibiotic agent or possibly show antibiotic resistance is important when deciding on the treatment of the patient. Currently, the corresponding clinical evaluation from patient samples requires bacterial culturing for up to several days. In the case of high risk patients this period is often bridged by applying broad-spectrum antibiotic treatments. This is not only costly and inefficient but also itself accelerates the problem of antibiotic-resistant bacteria. In the methods currently used for bacterial culturing the work flow is not automated, nor are the results 100 % accurate. Alternative solutions to the foregoing problem would therefore be desirable and also are of significant commercial interest.

For a more rapid detection or evaluation of biological material, like bacteria or free DNA, in samples it would be necessary or important to enrich the biological material, e.g. bacteria, for carrying out a subsequent test or evaluation step. One especially important step in the future diagnosis of infectious diseases refers to the quick and efficient enrichment of bacteria or bacterial concentration in a sample.

A method for microbial detection and antimicrobial susceptibility testing is disclosed in WO 2006/066216. The methods described therein comprise the enrichment and localization of bacteria at a surface, and subsequently detecting and/or testing the bacteria. The method described in WO 2006/066216, requires the use of redox mediators which may harm or even destroy the biological material in a sample.

### SUMMARY OF THE INVENTION

The present invention is directed to a method and a device for the enrichment of biological material.

In a first aspect of the invention, a method for the enrichment of biological material in a sample is provided. The method comprises the steps of providing a microfluidic device having at least one conducting polymer electrode; adding a sample containing biological material and an electrolyte to said device having at least one electrode; and applying an electrical voltage to the electrode(s), and thus enriching the biological material.

According to a preferred embodiment of the inventive method, at least two electrodes are used, and an opposite electrical charge is applied to said at least two electrodes. It is further preferred, that said at least two electrodes are arranged at opposing sides of a fluidic volume being contained in the device, or that said at least two electrodes are arranged adjacent to each other.

In another preferred embodiment of the first aspect of the invention, said at least one polymer electrode is provided on a substrate. Further preferably, said electrolyte comprises de-ionized water, and said electrolyte further contains at least one of a buffering agent, an osmolarity increasing agent, and a growth medium.

In still another embodiment of the first aspect of the invention, said substrate is selected from the group consisting of glass, plastic, resins, metal, metal alloys, and paper. According to yet another embodiment of the first aspect of the invention, said electrode comprises a polymeric material selected from the group consisting of poly(acetylene)s, poly(pyrrole)s, poly(thiophene)s, poly(aniline)s, poly(fluorene)s, poly(3-alkyl-thiophene)s, polytetrathiafulvalenes, polynaphthalenes, poly(p-phenylene sulfide)s, poly(p-phenylene vinylene)s, and derivatives thereof.

In a still further embodiment of the first aspect of the invention, said electrolyte is a low conductivity electrolyte, preferably an electrolyte having a conductivity of less than 1 S/m.

According to a preferred embodiment of the invention, said biological material is selected from the group consisting of bacteria, free DNA, amino acids, proteins, fungi, algae, and protozoa, including viruses, prions and other pathogens.

In another preferred embodiment of the inventive method, said biological material is a charged biological material, or is charged *in-situ* in the electrolyte.

A second aspect of the invention is directed to the use of a conducting polymer electrode for the enrichment of biological material in a sample.

Finally, a third aspect of the invention is directed to a microfluidic device comprising an enrichment area for enriching biological material, wherein said enrichment area comprises a conducting polymer electrode.

According to a preferred embodiment of the third aspect of the invention, the conducting polymer electrode is a conjugated polymer electrode, and/or said conducting polymer is an ion-conducting polymer containing an electrochemically and an electronically conducting species.

### BRIEF DESCRIPTION OF THE DRAWINGS

- Fig. 1: shows a microfluidic device according to an embodiment of the invention.
- Fig. 2: shows a microfluidic device according to another embodiment of the invention.
- Fig. 3: shows a microfluidic device according to yet another embodiment of the invention.
- Fig. 4: shows the microfluidic device of Example 1.
- Fig. 5: shows the microfluidic device of Example 2.
- Fig. 6: shows the relationship between applied voltage and enrichment of bacteria in Example 3.

### DETAILED DESCRIPTION OF EMBODIMENTS

The present invention refers to a method for the enrichment of biological material. The method comprises the steps of providing a microfluidic device having at least one conducting polymer electrode; adding a sample of biological material and an electrolyte to the fluidic volume of said device and applying an electrical voltage to the at least one electrode in order to start the enriching of the biological material.

The inventive method is based on the use of a conducting polymer electrode for the generation of an electric field, which is used for enriching biological material by this electric field. Accordingly, the inventive method requires at least one conducting polymer electrode. Sometimes these electrodes are also referred to as conducting polymeric electrodes.

A "conducting polymer electrode" in the meaning of the present invention consists essentially of a polymer material. This material can be a matrix wherein conducting material is introduced, like conducting metal particles. Preferably, the conducting polymer electrode is a conjugated polymer electrode, also known as an intrinsically conducting polymer electrode. A conjugated polymer usually has a conjugated backbone having a delocalized electron system. Such delocalized electron system can be obtained by a molecular structure having alternating single and double bonds. In an especially preferred embodiment, the electrode consists of a conjugated polymer.

The polymer electrodes to be used according to the present invention are also electrochemically conducting. Within the electrode, ionic species can be generated when the polymer is oxidized or reduced. When the electrode is in contact with an electrolyte containing ionic species, some of the ionic species of the electrolyte can migrate from the electrolyte into the polymer electrode. The inverse process, i.e. the migration of an ionic species from the polymer electrode to the electrolyte, can also occur at the interface between the polymer electrode and the electrolyte. The latter process compensates the charge difference between the electrode and the electrolyte, and this process thus corresponds to an electrochemical conduction between the electrolyte and the electrode.

Exemplarily, the foregoing processes can be explained or illustrated with a PEDOT:PSS electrode. When a DC-potential (direct current potential) is applied between two electrodes, an electric field will be created perpendicular to the flow direction in the channel. If the biological sample to be enriched contains ions, like cations, the oxidation and reduction of the electrodes takes place inside the electrodes according to the following reaction (M⁺ denotes cations and e⁻ electrons):

PEDOT⁺PSS- + e⁻ + M⁺ → PEDOT⁰ + PSS⁻M⁺

The reduction (left to right in the reaction above) takes place at the negatively biased electrode and oxidation (opposite reaction, i.e. right to left) at the positive electrode.

As the polymer electrode is capable of both an electrochemical conduction, i.e. by migration of ionic species, and an electronic conduction, i.e. by conducting electrons, a conductive pathway from the electrode to the electrolyte, and vice versa, can be provided.

In contrast thereto, the conduction at the interface of a conventional electrode and an electrolyte always comprises or requires a redox reaction. A current can only be established between an electrolyte and an electrode by generating or consuming ionic species. Such redox process, however, is accompanied by undesired side reactions, like the decomposition of water. Such side reactions can hardly be avoided, and the number and intensity of side reactions increase with increasing potential difference between the electrode and the electrolyte. The species generated by undesired side reactions, i.e. the by-products, may be detrimental to the electrode, the electrolyte, or even to the biological material. Usually redox agents are added to the electrolyte to avoid such side reaction, or to control the side reactions and the corresponding by-products. However, also redox agents may have detrimental effects on the device or on the biological material itself.

With the use of polymer electrodes according to the present invention, the addition of redox agents may be reduced, or even omitted completely. Thus, in a preferred embodiment of the invention, the electrolyte and the sample are substantially free of any oxidizing and/or reducing agent, i.e. the electrolyte and the sample contain no oxidizing and/or reducing agent.

Further, the use of polymer electrodes is also beneficial for detecting or analyzing biological material in a sample or on the electrode itself. As polymer electrodes can be transparent to analyzing radiation, the biological material can be analyzed through the electrode, e.g. by UV-, visible, or IR-analytical instruments, or by other optical instruments like a microscope.

Another aspect of the present invention refers to a microfluidic device comprising an enrichment area for enriching biological material, wherein said enrichment area comprises at least one conducting polymer electrode. In the following, when reference is made to the inventive method for the enrichment of biological material, it is to be understood that the corresponding embodiments also can be used for the inventive microfluidic device.

A microfluidic device in the meaning of the present invention is a device which can be used for microfluidic technologies. The microfluidic device can be operated with low sample and reagent volumes, like volumes in the range of 10 µl to 100 ml, preferably 50 µl to 10 ml. The term "fluidic volume" refers to the volume provided by the microfluidic device which can be filled with the electrolyte and the sample.

The term "enrich" or "enrichment" is used in this application to express an increase in concentration. This increase in concentration can be locally, i.e. in some portions or regions of the sample the concentration of biological material is increased, and in other regions the concentration is consequently decreased. The latter corresponds to a depletion of biological material in this portion of the sample. The enrichment of biological material can be achieved in a flowing stream of a sample, wherein the flow is separated into an enriched sub-stream and a depleted sub-stream, or the biological material is enriched on or close to an electrode. The enrichment on the electrode can be achieved by attaching the biological material to the surface of the electrode, and the biological material is immobilized on the electrode for further analysis or further studies. The flow of the sample is not necessary for the enrichment of biological material on or close to an electrode.

The principle underlying the method according to the present invention refers to the enrichment or concentration of charged biological material in an electric field using at least one polymeric electrode. The charged biological material is drawn by the electric field towards the electrode, or is pushed away from the electrode, depending on the charge of the biological material and voltage polarity on the electrode. If the biological material is charged with a charge of the same sign as the polarity of the electrode, it is repelled from the electrode. On the other hand, if the biological material is charged with a charge in the opposite sign, it is attracted by the electrode. In this context, it is preferred, that the biological material and the at least one electrode are charged at different signs so that the biological material is attracted by the electrode.

In the case that only one electrode is used, this electrode must be charged by a positive or negative charge. If two electrodes are used, both electrodes are preferably charged at different signs in order to establish a potential difference between these two electrodes, thus establishing an electric field gradient between the two electrodes. This electric field is used for collecting or enriching the charged biological material at one electrode. The electrodes can be setup to establish a single electric field, multiple electric fields, or even a sequential electric field or a field gradient.

The enrichment or concentration of the biological material can be achieved in different ways. According to a preferred embodiment of the invention, a flow of biological material in an electrolyte is passed through an electric field established at said at least one electrode. As the biological material enters the electric field, the biological material is influenced by the electric field, and consequently the biological material is enriched at one of the electrodes. The part of the sample containing the enriched biological material can be separated from the depleted part of the sample.

In another preferred embodiment of the invention, the biological material is enriched and localized on or close to a polymer electrode. With the localization of the biological material on or close to the electrode, the material can be analyzed, or further processed as outlined in WO 2006/066216, the analyzing and testing methods of which are incorporated herein explicitly by reference.

The biological material to be enriched according to the method of the invention is part of a sample. The sample may contain a single biological material, or several different species of biological material. Typical samples according to the present invention are body fluids, like blood and urine samples. However, all other bodily fluids can also be used according to the present invention. Additionally, the invention can also be used for environmental samples, like samples of lakes and rivers, or food samples, like milk and juices. If a liquid sample is not readily available, the bacteria present can also be brought into liquid form by washing the bacteria from a surface, or by providing a smear from a bacteria containing surface and suspending the collected bacteria in a sample liquid, like an electrolyte. Thus, the sample may further contain an electrolyte. Alternatively, the sample and the electrolyte can be added separately to the fluidic device.

The biological material used in the method has to have a charge. This charge can be the natural or intrinsic charge, or the biological material can be charged prior to adding to the device or, in the alternative, within the device (in-situ). The biological material can be of various type. Most bacteria, the preferred biological material, are naturally negatively charged. The same applies to most human cells or free DNA, which can both be used as biological material according to the present invention. Also, proteins or amino acids can be charged negatively or positively, depending on the pH of the electrolyte.

In some cases it is also possible to use uncharged biological material and charge it prior to the addition to the electrolyte, or *in-situ* within the electrolyte. For example, the charge can be applied using an oxidation or reduction reaction. This oxidation or reduction reaction can take place on or close to the electrode using the electrode to generate the charge, or with the addition of an oxidizing or reducing agent. In an alternative embodiment of the invention, the pH of the electrolyte can be changed to a desired value or range, resulting in a charge of the biological material. The change of pH can, for instance, be achieved by an electrochemical reaction producing or consuming protons, or by loading the electrode with protons which can be expelled into the electrolyte when a potential is applied to the electrode. Alternatively, the pH of the electrolyte can be adjusted to a desired value or range using commonly known buffers, as exemplified below.

An essential element of the method and the device according to the present invention is the use of a polymer electrode. In a preferred embodiment, the electrode comprises a polymeric material selected from the group comprising poly(acetylene)s, poly(pyrrole)s, poly(thiophene)s, poly(aniline)s, poly(fluorene)s, poly(3-alkylthiophene)s, polytetrathiafulvalenes, polynaphthalenes, poly(p-phenylene sulfide), poly(p-phenylene vinylene)s, and derivatives thereof. Preferably, the electrode comprises at least two different polymeric materials selected from the above given group. Specifically preferred polymer combinations for the use as polymer electrodes are poly(3,4-ethylene dioxythiophene) : polystyrene sulfonate, polypyrrole : polystyrene, polyaniline : polystyrene sulfonate, poly(3,4-ethylene dioxythiophene) : dodecylbenzenesulfonate, polypyrrole : dodecylbenzenesulfonate. However, other combinations are also encompassed.

The conductivity of an electrode is usually given as the sheet conductivity of the polymer electrode. In a preferred embodiment of the invention, the sheet conductivity of the electrode is less than 1000 Ω per square, preferably less than 500 Ω per square, further preferably less than 350 Ω per square, the latter corresponding to a material resistivity of 70 µΩm.

The electrolytes used in the present invention are preferably low conductivity electrolytes. In a preferred embodiment, the electrolyte has a conductivity of less than 1 S/m, preferably less than 0.5 S/m, more preferably less than 0.1 S/m, even more preferably less than 0.03 S/m, and most preferably less than 0.005 S/m.

The voltage applied to the electrodes is preferably a low DC potential. With a low potential, the decomposition of the sample, especially the electrolyte, or the destruction of the electrode can be minimized or even avoided. In a preferred embodiment, the applied voltage is below 5 V, preferably below 3 V, further preferably below 2 V, even further preferably below 1 V, and most preferably below 500 mV.

The use of polymer electrodes gives the possibility to use very low potentials for enrichment of biological samples. The application of low potentials has several advantages. First, when using low potentials, like potentials below 500 mV, the risk of water electrolysis or breakdown of the electrodes is diminished. Also, for applications where the bacteria are enriched for use in phenotypic studies or viability checks, such low potentials have little effect on the well-being of the bacterial cells. Finally, the utilization of low voltages and low currents makes the device power efficient and therefore also allows for integrating of 'low performance' power sources, such as printed batteries.

In another preferred embodiment of the inventive method or the inventive device, the size of the electrodes is larger than the area contacted by the sample in order to avoid or minimize the over-oxidation of the electrode, thus making sure that there is enough polymeric material to be oxidized/reduced. The same effect can also be achieved by making the electrode thicker. In a preferred embodiment of the invention, the area contacted by the sample is less than 10 % of the total area of the polymer electrode, preferably less than 5 %, more preferably less than 2 %, and even more preferably less than 1%.

The use of polymer electrodes further has the advantage of being readily available, easily processable, cheap and biocompatible.

The polymer electrodes used in the present invention may be incorporated into a microfluidic device. For the use of the polymer electrode in such devices, the polymer electrodes are usually applied to a substrate. The substrate can be any suitable substrate, like a glass or plastic substrate. Preferably, a transparent substrate is used. This allows for the use of optical detection and analyzing methods. Thus, the biological material enriched or even localized on the surface of the electrode can be detected and analyzed through the substrate.

In a preferred embodiment of the present invention, the substrate can be selected from a wide variety of materials, including, but not limited to, silicon such as silicon wafers, silicon dioxide, silicon nitride, glass and fused silica, quartz, soda-lime glass, borosilicate glass, acrylic glass, sugar glass, isinglass or aluminium oxynitride, paper, ceramics, polyimide, plastics, resins and polymers including polymethylmethacrylate, acrylics, polyethylene, polypropylene, polyethylene terephthalate, polycarbonate, polystyrene and other styrene copolymers, polytetrafluoroethylene, metals and metal alloys, such as aluminum, steel, gold, silver, copper, tungsten, molybdenum, tantalum, brass, etc. High quality glasses such as high melting borosilicate or fused silicas may be preferred for their UV transmission properties. It is also possible to coat the device or substrate with a variety of coatings, e.g. to reduce non-specific binding, introduce biocompatibility, or to influence the flow resistance.

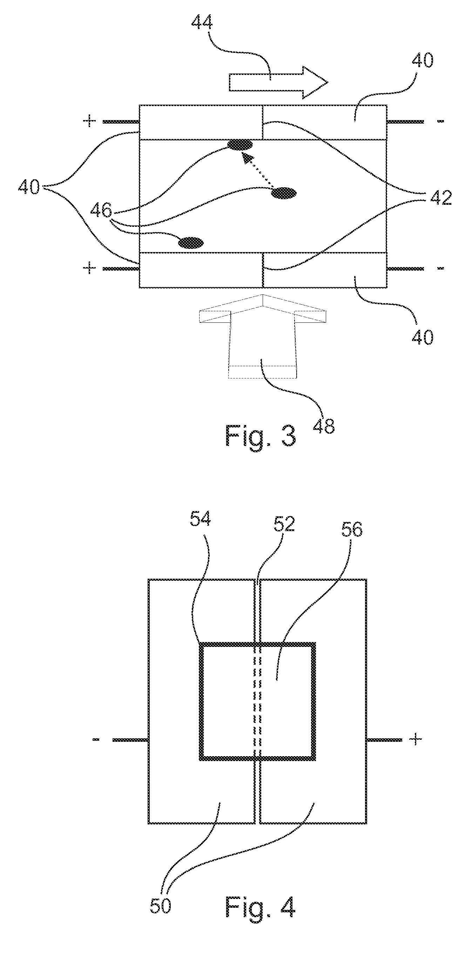

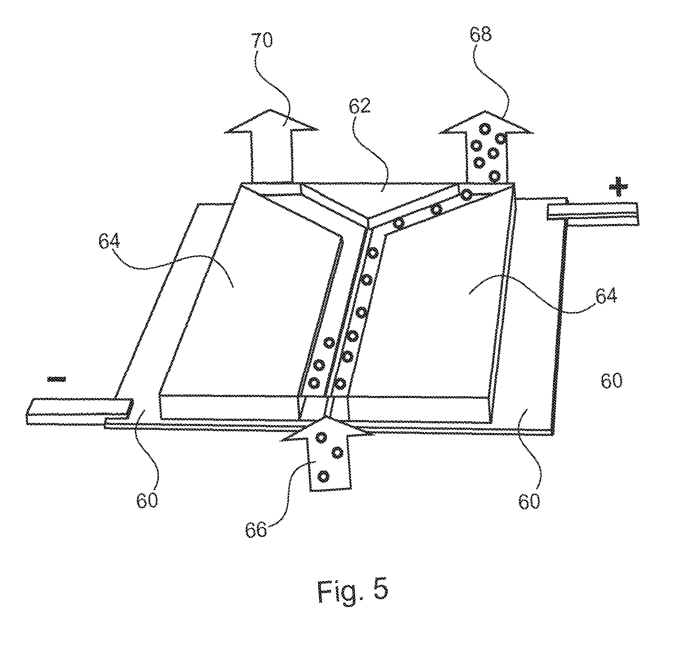

The device can be designed in a variety of designs. Possible designs are described in WO 2006/066216 and are hereby incorporated by reference. Further possible embodiments or designs are also shown in Figures 4 and 5.

The electrolytes of the present invention are preferably based on a solvent. In a preferred embodiment of the invention, the solvent is selected from water, de-ionized water, alcohols, like methanol or ethanol, glycerol, or mixtures thereof. In another preferred embodiment, the electrolyte further contains at least one of a buffering agent, an osmolarity increasing agent, and a growth medium.

The buffering agent may be used to adjust the pH of the electrolyte, and is preferably selected from 4-(2-hydroxyethyl)-1-piperazineethanesulfonic acid (HEPES), 3-[4-(2-hydroxyethyl)-1-piperazinyl]propanesulfonic acid (HEPPS), N-cyclohexyl-3-aminopropanesulfonic acid (CAPS), 3-(cyclohexylamino)-2-hydroxyl-1-propanesulphonic (CAPSO), N-cyclohexyl-2-aminoethanesulfonic acid (CHES), 3-(N-morpholino)propanesulfonic acid (MOPS), 2-(N-morpholino)ethanesulfonic acid (MES), and tris(hydroxymethyl)aminomethane (TRIS). It is also possible to use a combination of different buffering agent for adjusting the pH of the electrolyte.

The osmolarity increasing agent may be used for increasing or adjusting the osmolarity of the electrolyte. Preferably, the osmolarity increasing agent is a sugar or a zwitter-ionic species. In a preferred embodiment of the invention, the osmolarity increasing agent is selected from g!ycylgiycme, glucose, sucrose, trehalose, succinate, cyclodextrin, arginine, galactose, mannose, maltose, mannitol, glycine, lysine, citrate, sorbitol, dextran, sodium chloride, and combinations thereof. Most preferably, the osmolarity increasing agent is sucrose.

Additionally, the electrolyte may contain a growth medium supporting the growth of e.g. bacteria in the electrolyte. The growth medium may be any suitable salt, or any other nutrient, known to a person skilled in the art.

The biological material which can be enriched or concentrated according to the present invention can be any material having a charge intrinsically, or which can be charged. In a preferred embodiment, the biological material is selected from bacteria, free DNA, amino acids, proteins, fungi, algae, and protozoa, and can include viruses, prions or other pathogens.

The bacteria may be selected from genera such as Bacillus, Vibrio, Escherichia, Shigella, Salmonella, Mycobacterium, Clostridium, Cornyebacterium, Streptococcus, Staphylococcus, Haemophilus, Neisseria, Yersinia, Pseudomonas, Chlamydia, Bordetella, Treponema, Stenotrophomonas, Acinetobacter, Enterobacter, Klebsiella, Proteus, Serratia, Citrobacter, Enterococcus, Legionella, Mycoplasma, Chlamydophila, Moraxella, and Morganella. Similarly, the fungi may be selected from genera such as Candida, and Aspergillus. The viruses may comprise orthomyxoviruses, (e.g. influenza virus), paramyxoviruses (e.g. respiratory syncytial virus, mumps virus, measles virus), adenoviruses, rhinoviruses, coronaviruses, reoviruses, togaviruses (e.g. rubella virus), parvoviruses, poxviruses (e.g. variola virus, vaccinia virus), enteroviruses (e.g. poliovirus, coxsackievirus), hepatitis viruses (including A, B and C), herpesviruses (e.g. Herpes simplex virus, varicella-zoster virus, cytomegalovirus, Epstein-Barr virus), rotaviruses, Norwalk viruses, hantavirus, arenavirus, rhabdovirus (e.g. rabies virus), retroviruses (including HIV, HTLV-I and II), papovaviruses (e.g. papillomavirus), polyomaviruses, and picornaviruses, and the like.

The bacteria may also be defined according to their reactivity, and may be selected from the group of gram-positive cocci, gram-positive bacilli, gram-negative cocci, gram-negative bacilli, or bacteria which are not possible to gram stain. Examples of gram-positive bacilli are Clostridium difficile, Bacillus anthracis or Bacillus cereus. Gram-positive cocci can be e.g. Staphylococcus aureus, Streptococcus pneumoniae or Staphylococcus epidermidis. Gram-negative bacilli are e.g. Escherichia coli, Legionella pneumophila or Pseudomonas aeruginosa. Gram-negative cocci are e.g. Neisseria gonorrhoeae, Neisseria meningitides or Moraxella catarrhalis.

If different species of biological material are present at the same time within a sample, these species might disturb each other and the process of enrichment. There are various methods of separating or pre-processing of the sample prior to or while applying the method of the present invention. According to one embodiment, the surface of the electrodes might be coated with antibodies. In a first step, the Electric field can be used to pull the biological material towards the electrode. Once the biological material is on the surface, the polarity of the Electric field is reversed. Only those species of biological material with certain membrane proteins will stay on the surface, while others can be washed away.

Alternatively, a pH gradient can be created using the Electric field and the electrode, as outlined above. A separation on the isoelectric response can then be carried out e.g. for separating different biological materials.

Finally, according to another embodiment, the method of the present invention can be used to separate different biological material by applying an electric field perpendicular to the flow direction of the sample. With a flow perpendicular to the Electric field, species of biological material will have different trajectories depending on the charge of the species. These species can then be collected at different outputs and are differentiated by their charge.

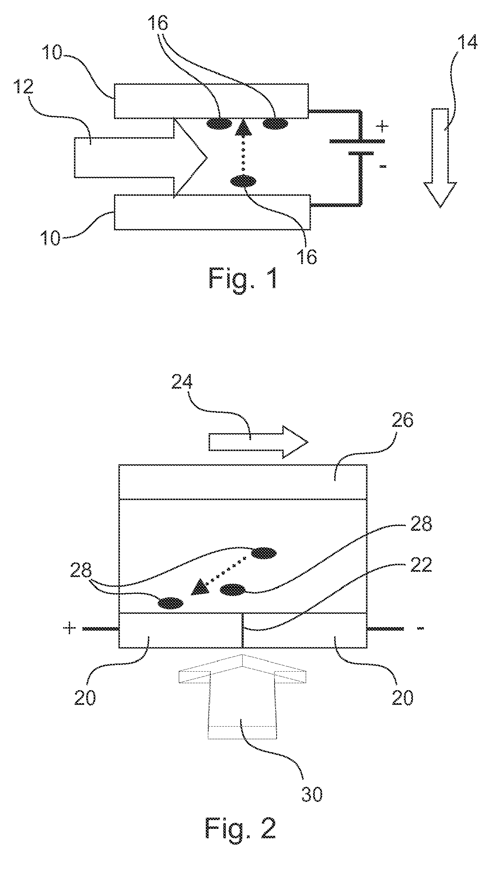

The polymer electrodes used in the present invention can be arranged in different alignments. According to a first embodiment of the invention, the electrodes are arranged in an opposite arrangement with a flow of the sample perpendicular to the electric field between the electrodes, as can be seen from Fig. 1. Polymer electrodes 10 are arranged on opposing sides of a flow channel, with the flow of the sample from the left to the right. The electric field 14 is built up from top to bottom perpendicular to the flow direction of the sample. Negatively charged bacteria 16 are attracted by the positively charged electrode.

In another preferred embodiment, the polymer electrodes can be arranged adjacent to each other. As can be seen from Fig. 2, polymer electrodes 20 are arranged adjacent to each other, separated by an insulating line 22. The electric field 24 is built up from left to right. The flow channel is covered with transparent glass cover 26. Negatively charged bacteria 28 are directed towards the positively charged electrode in a flow as indicated by flow direction 30. Oxidation takes place at the positively charged electrode, and reduction at the negatively charged electrode.

In still another embodiment, adjacent electrodes can be arranged on opposing sides of a flow channel, as illustrated in Fig. 3. Adjacent polymer electrodes 40 are separated by insulating line 42. The polymer electrodes 40 form the top and bottom of a flow channel. However, it is also possible that opposing electrodes 40 form left and right walls of a flow channel. An electric field 44 is built up from left to right, attracting negatively charged bacteria 46 to the positively charged electrode. The flow of the sample is indicated by flow direction 48.

In the following, the invention is exemplified by examples. These examples should not be construed as limiting the present invention.

### EXAMPLES

### Example 1

To test the electrophoretic capture of biological species, two-electrode structures were made using commercial PEDOT:PSS pre-coated on a plastic foil (Orgacon EL-350, Afga-Gevaert), as schematically shown in Fig. 4. The conducting polymer 50 was divided into two electrodes, separated by an insulating line 52. The electrodes were covered by a 10 mm x 10 mm x 0.25 mm double-sided tape frame 54.

E coli K12 was cultured over night in Brain-Heart-Infusion to a concentration of roughly 10⁹ cfu/ml. Thereafter the bacteria where centrifuged with a microcentrifuge at 10 000 rpm for 3 min. The bacterial pellet was re-suspended in de-ionized water and the washing repeated. In an alternative experiment, the bacterial pellet was re-suspended in a low conductivity sucrose buffer (300 mM sucrose and 10 mM HEPES). For fluorescence experiments, the bacteria where stained with the Invitrogen LIVE/DEAD BacLight stain for 15 min before the first washing step.

The sample solution 56 was pipetted into the frame 54 and sealed with a microscope cover glass. Voltages were applied with a Keithley 2402 source meter and the maximum current was limited to 105 µA. When a DC-potential of 1 or 2 V is applied, the E coli move towards the positive electrode. Depleted regions on the reduced side and enriched regions on the oxidized side could be observed.

### Example 2

In order to show the usability of conjugated polymers for sample enrichment in Lab-on-a-chip devices, the tape frame of Example 1 was replaced by a Y-shaped microfluidic channel 64 on top of two PEDOT:PSS electrodes, as schematically shown in Fig. 5. Since the device is symmetrical with the same electrochemically active electrode material 60 on either side of the insulating line 62, there is no threshold potential that has to be exceeded in order to create an electric field. Naturally, the electrode material 60 itself has a limited conductivity (350 Ω/square) but due to the very high resistance of the insulating line 62 and the low conductivity of the medium, the entire potential drop will still be located between the electrodes.

The microfluidic chip was connected to a flow pump (Harvard apparatus) with standard microfluidic connectors The devices was loaded with 25 µl sample solution and sealed with a microscope cover glass. Voltages where applied with a Keithley 2402 sourcemeter, connected to PEDOT:PSS film with crocodile clamps. On outlet 68 at the positive electrode, an enriched sample could be collected, while the sample collected at outlet 70 was depleted from the bacteria.

### Example 3

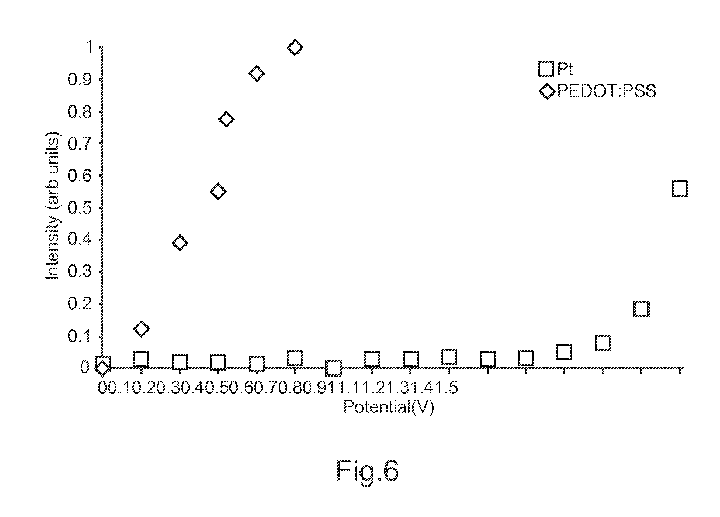

To determine the correlation between the applied potential and the enrichment of the sample, a setup as in Example 2 is supplied with a sample liquid. The biological material is represented by fluorescent carboxylated (i.e. negatively charged) 2 µm latex beads. The sample liquid, beads in water, is flowed through the device at 20 µl/min and the fluorescence in the channel region on top of the positive electrode is measured about 15 mm downstream from the sample inlet. Fig. 6 shows the relationship between applied voltage and enrichment within the channel, in this case measured as the normalized fluorescence of the fluorescent latex beads. The measurement points in Figure 6 correspond to the steady-state intensity, when the current has declined to a stable value (here, about 0.3 µA/V). As a comparison, the same measurement can also be performed on a device with platinum electrodes. This comparison shows the advantageous behavior of the polymer electrode in view of a Pt-electrode.

As seen from Figure 6, less than 100 mV is enough to move the beads or other negatively charged particles, towards the positive electrode. Within the given potential range, 0 - 500 mV, the effect scales linearly with the voltage.

### LIST OF REFERENCE SIGNS

- 10: polymer electrodes
- 12: sample flow direction
- 14: electric field direction
- 16: negatively charged bacteria
- 20: polymer electrodes
- 22: insulating line
- 24: electric field direction
- 26: glass cover
- 28: negatively charged bacteria
- 30: sample flow direction
- 40: polymer electrodes
- 42: insulating line
- 44: electric field direction
- 46: negatively charged bacteria
- 48: sample flow direction
- 50: polymer electrodes
- 52: insulating line
- 54: tape frame
- 56: sample
- 60: polymer electrodes
- 62: isolation
- 64: Y-shaped microfluidic channel
- 66: sample inlet
- 68: enriched sample outlet
- 70: waste outlet

## Claims

1. A method for the enrichment of biological material comprising the steps of
- providing a microfluidic device having at least one conducting polymer electrode (10;20;40;50;60);
- adding a sample (56) containing biological material (16;28;46) and an electrolyte to said device having said at least one electrode (10;20;40;50;60);
- applying an electrical voltage to the at least one electrode (10;20;40;50;60);
- and enriching the biological material (16;28;46).

2. Method according to claim 1, wherein at least two electrodes (10;20;40;50;60) are used, and an opposite electrical charge is applied to said at least two electrodes (10;20;40;50;60).

3. Method according to claim 2, wherein said at least two electrodes (10;20;40;50;60) are arranged at opposing sides of the device, or wherein said at least two electrodes (10;20;40;50;60) are arranged adjacent to each other.

4. Method according to any of the preceding claims, wherein said at least one polymer electrode (10;20;40;50;60) is provided on a substrate.

5. Method according to claim 4, wherein said substrate is selected from the group consisting of glass, plastic, resins, metal, metal alloys, and paper.

6. Method according to any of the preceding claims, wherein said electrode (10;20;40;50;60) comprises a polymeric material selected from the group consisting of poly(acetylene)s, poly(pyrrole)s, poly(thiophene)s, poly(aniline)s, poly(fluorene)s, poly(3-alkylthiophene)s, polytetrathiafulvalenes, polynaphthalenes, poly(p-phenylene sulfide)s, poly(p-phenylene vinylene)s and derivatives thereof.

7. Method according to any of the preceding claims, wherein said electrolyte is a low conductivity electrolyte, preferably an electrolyte having a conductivity of less than 1 S/m.

8. Method according to claim 7, wherein said electrolyte comprises de-ionized water, and wherein the electrolyte further contains at least one of a buffering agent, an osmolarity increasing agent, and a growth medium.

9. Method according to any of the preceding claims, wherein said biological material (16;28;46) is selected from the group consisting of bacteria, free DNA, amino acids, proteins, fungi, algae, and protozoa, including viruses, prions and other pathogens.

10. Method according to any of the preceding claims, wherein said biological material (16;28;46) is a charged biological material, or wherein the biological material (16;28;46) is charged *in*-*situ* in the electrolyte.

11. Use of a conducting polymer electrode (10;20;40;50;60) for the enrichment of biological material (16;28;46) in a sample.

12. A microfluidic device comprising an enrichment area for enriching biological material, wherein said enrichment area comprises at least one conducting polymer electrode (10;20;40;50;60).

13. Microfluidic device according to claim 12, wherein said conducting polymer is a conjugated polymer.

14. Microfluidic device according to claim 12 or 13, wherein said conducting polymer is an ion-conducting polymer containing an electrochemically and an electronically conducting species.

15. Microfluidic device according to claims 12, 13 or 14 wherein said electrode (10;20;40;50;60) comprises a polymeric material selected from the group consisting of poly(acetylene)s, poly(pyrrole)s, poly(thiophene)s, poly(aniline)s, poly(fluorene)s, poly(3-alkylthiophene)s, polytetrathiafulvalenes, polynaphthalenes, poly(p-phenylene sulfide)s, poly(p-phenylene vinylene)s and derivatives thereof.
